# EUROPEAN PATENT SPECIFICATION

(11) **EP 1 836 181 B1**
(45) Date of publication and mention of the grant of the patent: **11.03.2009**
(21) Application number: 04762306.1
(22) Date of filing: 31.08.2004
(51) Int. Cl.: C07D 307/50, D21C 3/24, C13K 1/02, C12P 7/10

(54) **METHOD AND DEVICES FOR THE CONTINUOUS PROCESSING OF RENEWABLE RAW MATERIALS**
VERFAHREN UND VORRICHTUNGEN ZUR KONTINUIERLICHEN VERARBEITUNG ERNEUERBARER ROHSTOFFE
PROCEDE ET DISPOSITIFS DE TRAITEMENT CONTINU DE MATIERES PREMIERES RENOUVELABLES

(43) Date of publication of application: 26.09.2007
(73) Proprietor: Biomass Technology Ltd., 1097 Nicosia (CY)
(72) Inventor: KRATOCHVÍL, Zdenek, 162 00 Praha 6 (CZ); BOUSKA, Frantisek, 150 00 Praha 5 (CZ); MACHEK, Frantisek, 158 00 Praha 5 (CZ)
(74) Representative: Danek, Vilém
(86) International application number: PCT/CZ2004/000054
(87) International publication number: WO 2006/024242

(56) References cited:
- EP-A- 1 130 085
- WO-A-96/25553
- US-A- 4 564 595
- US-A1- 2002 197 686

## Description

### Field of the invention

The invention relates the method of processing lignocellulosic materials using continuous pressure hydrolysis resulting to the production of furfural and furane, acetic acid, lignin, hydrolytic sugars - glucose. The method also includes processing the residues after hydrolysis using methods of enzymatic hydrolysis, hydrolysis of starch from added starch materials, esp. cereal grains to glucose, its fermentation, distillation and rectification to bioethanol, use of stillage by anaerobic fermentation to biogas, and accumulation of carbon dioxide, and the complex device for the carying of this method.

### Background of the invention

Deficiency in fossil raw materials is becoming a potential barrier inhibiting the economic and social development of most regions. Current systems for the production of organic chemicals are predominantly based on the use of fossilised raw materials. Fossilised raw materials, esp. crude oil and natural gas; are gradually being exhausted. The Czech Republic strongly depends on the import of the above mentioned raw materials, which is the cause of a considerable proportion of the deficit in the trade balance.

In addition to fossil resources, a big part of the renewable organic material - biomass - is available for the present and the future.

Biomass (LCM - lignocellulosic materials) is at present the most widely-used renewable resource and has an appreciable share of world production.

In the foreseeable future it will be necessary to search for and develop cheaper and more efficient methods of conversion to fine gaseous and particularly liquid fuels, which will allow wider and more efficient utilization of the biomass and the renewable resources as a whole, which means without restricting the operation to the proximity of the raw material resources, and also greater flexibility in the methods of application and without seasonal swings.

Sources from basic agricultural industry (various kinds of straw) and waste from wood processing and the forestry industry are the main producers of the biomass.

Methods employing hydrolysis are under close examination. The very low price of the input lignocellulosic raw materials is one advantage of these methods.

Hydrolytic technologies providing solution of hydrolytic sugars (cellulosic and hemicellulosic) in the proper concentration and preferably using the continuous method have not yet been fully subjected.

All these facts are mentioned in Philip W. Madson's paper "Bio-ethanol Experiences in the USA" delivered at the European Conference on Bioethanol (held at Lissa - Holland, May 1990). He concludes that despite technological advances the production of bioethanol is at a break-even point, if not actually unprofitable.

The technologies so far utilized and designed for the production of ethanol as a fuel are mostly based on the utilization of starch materials, particularly corn and cereals.

A second strategy, nowadays actively pursued, is the utilization of lower cost inputs, such as cellulose, in order to reduce the costs of the raw material.

A known method is described in the US Patent No. 4,564,595, which concerns the acidic hydrolysis of pre-delignificated cellulose and the subsequent fermentation of the incurred elemental sugars (principally glucose).

The Japanese Patent No. 59048090 A entitled "Preparation of Fuel Alcohol" tries to eliminate the high energy intensity of the known methods. Its cornerstone is based on the idea that elemental sugars, using fermentation, are produced from renewable raw materials, and then fermented to ethanol. Starch materials are cracked by fibrous fungi of the Aspergilus genus, lignocellulosic materials such as wood are modified by yeast, straw and materials similar to straw are modified by Bacillus natto.

To produce furaldehyde, the Quaker Oats company uses discontinuous hydrolysis of lignocellulose with sulphuric acid (5% aqueous solution) at temperatures ranging from 145 to 170 °C .

The Swedish company Defibrator has elaborated a continuous hydrolysis, but they use a one-step expansion and before hydrolysis the raw material is impregnated by sulphuric acid.

A Finnish patent, registered in the Czech Rep. under No. 191945, the subject of protection of which is the method of two-step hydrolysis in the presence of sulphuric acid. In Step One, the hydrolysis is under way at temperatures from 150 to 200°C with the concentration of the sulphuric acid exceeding 10 % of the weight. In Step Two, at the same temperature the concentration of the acid is up to 5 % of the weight.

The Swiss patent registration CH 678183 A5 specifies the acidic hydrolyses of materials containing pentozans in the medium of 2 % weight of sulphuric acid at temperatures from 170 to 230°C.

Although the utilization of fossil raw materials is still economically more profitable, big petrochemical companies have established research and developmental workplaces focusing on new technologies utilizing renewable resources. The conference European on Bioethanol dealt with the causes of economic non-profitability from both the technological and legislative viewpoints. In the case of production of bioethanol, which is or is to be produced from maize or cereal starch, the sale price almost equals the price of the purchased raw material. These substrates are expensive but have the advatange of an easy and technologically subjected hydrolysis.

The European Patent No. 0 101 190 "Process for Producing Ethanol" by Assarsson and Nagasuy describes the production of glucosis subsequently condensed to ethanon utilizing the partial acidic hydrolysis of starch. Carbohydric material modified in various ways (chemically modified, derivative, unmodified, or mixture thereof) is considered to be the input raw material. In theory, cellulose also belongs to some of these groups although the authors do not expressly mention it in the list of raw materials. Nevertheless, the proposes conditions of the hydrolysis, esp. temperature, do not exclude it from the list of applicable materials. At the proposed heating steam temperature of 167 °C only the hydrolysis of a part of the pentozans can occur, but the lignocellulosic complex will remain intact. On that account, only starch material is mentioned in the requirements.

The continuous method of hydrolysis of lignocellulosic materials is not utilized industrially. In the operating conditions, if the process is to be economically bearable, it is difficult to observe the short reactive times, to ensure the fast heating of the mixture, as well as the heat regeneration and utilization percentage of all products. The lack of complexity in the utilization of products provided by a renewable material resource is another disadvantage of these methods.

The method for processing lignocellulosic materials using hydrolytic and fermentative procedures is partly dealt with by a number of patents (CZ 281504 and CZ PV 2000-4328).

The hydrolysis of lignocellulosic materials results in the production of furfural, acetic acid, lignin, hydrolytic sugars, esp. glucosis. According to the CZ patents, the nonhydrolysed residues of cellulose either return to the hydrolytic process, or go undergo extraction to acquire lignin. The disintegrated raw material is moistened at the weight ratio of 0,5 to 1:1, the redundant water is extracted from the acquired mixture, and subsequently pressure acidic hydrolysis is carried out. According to the preferred type of hydrolytic product, the hydrolytic process may be carried out at temperatures between 160 and 230 °C.

One disadvantage of this method is the high consumption of warm process water used to moisten the disintegrated lignocellulosic material and in its subsequent extraction before the hydrolysis itself. Another disadvantage of the methods designed so far is the fact that the energy saving of the whole operation has not yet been completely resolved.

The complex utilization of lignocellulosic and starchy materials has not yet been resolved either.

Ensuring the operating continuity of the production, particularly the supply of disintegrated lignocellulosic material to the hydrolyser, is an important condition for the processing of lignocellulosic materials during hydrolytic processes.

There is a filling piston unit consisting of a tank for material, two slide valves, and a filling and hydraulic cylinder. This unit does not ensure the fluent continuous dosing of lignocellulosic material and in additon to that this method of filling has not been tested for continuity of the hydrolytic process.

The filling press described in CZ 281504 consists of a cylindrical part and a conical part. Both parts are crossed by a worm with a constant pitch and a decreasing pitch in the conical part. The conical part consists of segments for conducting the liquid into the tank. The front face of the cylindrical part is perforated. At the end of the conical part there is a pressure filler, tightly connected with the filling press and running into the first hydrolyser. This filling system did not prove useful either, esp. because the filling was unequal and did not ensure the compactness and continuous clearness of the plug through the pressure filler. This system has been abandoned in particular for fear that it would not be suitable for commercial utilization in a non-stop operation.

### Brief summary of the invention

The aforementioned disadvantages of the existing hydrolytic methods for obtaining sugars - glucose, ethanol, furfural, pure lignin, biogas and carbon dioxide from lignocellulosic materials are resolved and eliminated by the method of complex processing of renewable raw materials, particularly lignocellulosic and starch materials. Method according to the invention is based on the following principle: the disintegrated lignocellulosic material moistured by process water at a ratio between 0,1 and 0,3 % of the weight, in relation to the weight of the input material, is continuously transported and by the mechanical heats moving materials to temperature 80 - 90°C in the filling unit. The disintegrated and heated material is further continuously hydrolysed in the presence of process water and steam at temperatures between 190 and 235 °C and at pressures of 1,5 to 3,2 MPa and in the presence of diluted acid in the quantity of 0,3 to 0,85 % of the weight, in relation to the suspension for a period of 9 to 12 minutes; the ratio of water and the solids matter is 1:3,5 to 1:4,5. The hydrolysis proceeds during the simultaous movement of the solid and liquid phase. The acid is dosed into the inlet piping in front of the hydrolyser. All the steam in the hydrolysing system will condense and heat the moving material. The released condensing heat will also compensate the heat loss through shell of the second hydrolyser. The catalytic effect of the acid and heat will cause the hydrolytic desintegration of the hemicelluloses to the furfural part and partly to hemicellulosic sugars. The lignocellulosic links will be disintegrated and cellulose degradates into glucose in the range from 27 to 85 %. When the hydrolysis is complete, suspension of the material is expanded in two stages. The vapour phase contains furfural, methanol, acetic acid and water. The hydrolysed suspension contains a solution of sugars, the residual solid defiberized lignocellulosic phase and water.

Continuous removal of the inert vapour phase containins predominantly furfural, out of the vapour area of the hydrolysers is important for the proper function and adjustment of the thermodynamic balance between the liquid and steam in the hydrolysers. Removal is carried out from the upper part of the hydrolysers and is important due to reaching the desired temperature. The pressure in the hydrolysers rises with the increasing content of this vapour phase.

The vapour phases from the hydrolysers and the vapour phases from the expanders are cooler and undergo rectification. They separate into a solution of furfufal, m ethanol, and water, and a mixture of acids and water. The furfural mixture is separated into two layers by decantation. The lower layer contains approximately 92 % w/w solution of furfural with water and methanol. The upper layer from the decanter contains about 8 % w/w furfural, further methanol and water. This layer is fed back into the rectifying column.

An azeotropic mixture of acids and water is extracted and a waterfree mixture of acetic acid and formic acid is obtained.

The hydrolysate containing hydrolysing sugars, the defiberized solid lignocellulosic phase, and water, is pressed. A solution of sugars and water and solid defiberized lignocellulosic residues, exposed to the activity of cellulolytic enzymes, is obtained by this pressuring. Lignocellulosic residues can also be returned to thermio - pressure hydrolysis for the completion of hydrolysis, or are extracted from the group consisting of ethanol or acetone by a solvent within 10 to 15 minutes. Lignin passes into the solvent and when the solvent evaporates, pure reactive lignin is obtained and the cellulose remains in the solid part. Pure glucose is obtained after cellulolytic hydrolysis. Sugars adsorbed to the fibrous material are pressed off into the sugar solution and do not return with the fibrous material into certain of the selected procedure of the solid residues processing. These easily combined procedural precautions secure a high yield of fermentable sugars.

The solution of glucose obtained by enzymatic hydrolysis is added to the sugar solution outgoing from the thermo-pressure hydrolysis and is mixed continuously with a starch material. Then everything undergoes amylolytic hydrolysis. The remaining solid particles, containing a non-starch proportion of grains, separated f rom the resulting reactive mixture and are returned to the thermo-pressure hydrolysis. The obtained glucose solution - after regulation of the pH, the addition of salts and nutrients and adjustment of glucose concentration by non-concentrated stillage from the mash column is filled via the heat exchanger to the fermentor. The spirituous fermentation is feeding with returning separated yeast cells, or 20 to 30 % of the fermentor's content is held in the fermentor as a yeast starter for the next fermentation, Whereby a new fermentation rapid starts. When the glucose ferments through to ethanol and the yeast cells are separated, the solution from the fermentor is pumped for distillation. About 90 % of the 40 % ethanol leaves the distilling column in the form of vapour for rectification. Part of the stillage is returned to fermentation to dilute the sugar solution to desired concentration. The unused part of the stillage proceeds to the evaporator. In the evaporator the stillage can be concentrated to the required concentration of the solids. The whole process has a very high utilization percentage in industrial conditions and the distilling efficiency is about 99,5 %.

The heat energy of the hydrolysed solution coming from the thermic pressure hydrolysis is used to the starchliquefying added to the glucose solution, or to heat the process water or steam. After the starchliquefying and adjusting the temperature so as to be optimal for the action of thermically stable amylases, the saccharifying of starch into glucose occurs very quickly. The advantage of this method is the application of heat generated from the hydrolytic solution.

The heat energy from the glucose solution and the heat from the stillage are used to preheat the feed for the mash column. The heat energy of the exhaust water is used to improve the energy balance of the thermo- pressure hydrolysis.

The compactness and linkage of the connected processes, hydrolysis, fermentation, distillation and rectification, and the processes utilizing side products (lignin, furfural, stillage, yeast cells, and carbon dioxide), facilitate automation of the process and achievement of a wasteless system of processing renewable resources.

The complex solution for the utilization of lignocellulosic and starch raw materials facilitates the maximum utilization of all input raw materials and thermal energy.

The device used for this method consists of a crusher and a processing tank, a filling unit and a set of hydrolysers, the last of which is connected (via a high-pressure slide valve) to a medium-pressure expander, the lower part of which is connected (via a medium-pressure slide valve) by a pipe to the upper part of a low-pressure expander. Thelower part of the low-pressure expander is interconnected (via a rotary feeder) by a pipe to the stirred tank of the hydrolytic product. The tank is further interconnected to the separating device, the first collecting part of which is interconnected to the first tank for the solution of sugar hydrolysate Its second collecting part for the solid unreacted phase is interconnected to the second tank for the remaining lignocellulosic phase. The upper part of the medium-pressure expander and low-pressure expander is interconnected to the upper part of the furfural rectifying column and to the furfural tank. According to the invention, the continuous pressure worm filling unit consists of segments made up of a body with single-threaded conveyer worms positioned on the shaft. The set of segments is complemented by a head, inside which a geometric shape is adapted to the position of a mandrel screwed into the end of the shaft. At least one steam ring and an spacer is placed between the worms. The steam ring widens conically on the entering side of the raw material. In the position of the steam ring the inside part of the body is fitted with a filler shaped like a thin annular ring 3 to 6 mm wide. The output flange fitted with an outlet filler with the reducing part leading to the first hydrolyser is fastened to the head. The body of one of the segments is fitted with an first side opening for the input of the disintegrated raw material and another opening for injecting pressurized process water. Furthermore, a connecting board and bearings are placed in front of the first worm on the shaft. The shaft is connected to the driving aggregate. The first hydrolyser is also equipped with a steam supply with a low-concentrated acid and is connected to at least one other hydrolyser. The last hydrolyser is interconnected via a high-pressure slide valve to the medium-pressure expander, the lower part of which is interconnected via a medium-pressure slide valve to the upper part of the low-pressure expander. Its lower part is interconnected via the rotating feeder to the mixing tank of the hydrolytic product. The upper part of the medium-pressure expander is interconnected via piping to the first exchangers, second exchangers, and via the third tank to the upper part of the first rectifying column. The upper part of the first rectifying column is interconnected, via the third exchangers and the lower part of the decanter, to the fourth tank for furfural and the upper part of the decanter is connected via the fifth tank for the low-concentration furfural mixture back to the third tank. The second tank for the unreacted solid lignocellulosic residues is interconnected via piping to the enzymatic hydrolyser, which is interconnected to the device for preparation of enzymes and the separator for separating the glucose from the lignin. The separator is connected to the sixth lignin tank and to the seventh tank for the preparation of the fermenting medium. The first tank for the solution of sugar hydrolysate is connected to pressure reactors for starchliquefying, which are equipped with a feed of ground starch raw material. The pressure reactors are interconnected to the enzymatic starch hydrolysers, which are further interconnected via the fourth and fifth exchangers to the fermentors. Both fermentors are interconnected via the yeast cell separator to the fourth heat exchanger, which is connected to the distilling device. The distilling device is interconnected to the evaporator and the second ethanol rectifying column. The distilling device, evaporator, and the second rectifying column are connected with the heating unit. The rectifying column in the area of the exhaust water is connected with the eighth accumulative water tank, in which the process water is heated in the first exchangers for the boiler of the heating unit.

The expanders are shaped as like a cyclone separator and the medium-pressure slide valve enters the medium-pressure expander tangentially.

### Brief description of the drawings

Figure 1 is a diagram of the hydrolytic fermentative process for processing lignocellulosic and starch materials.
Figure 2 demonstrates the continuous pressure worm filling unit.

### List of Related Captions

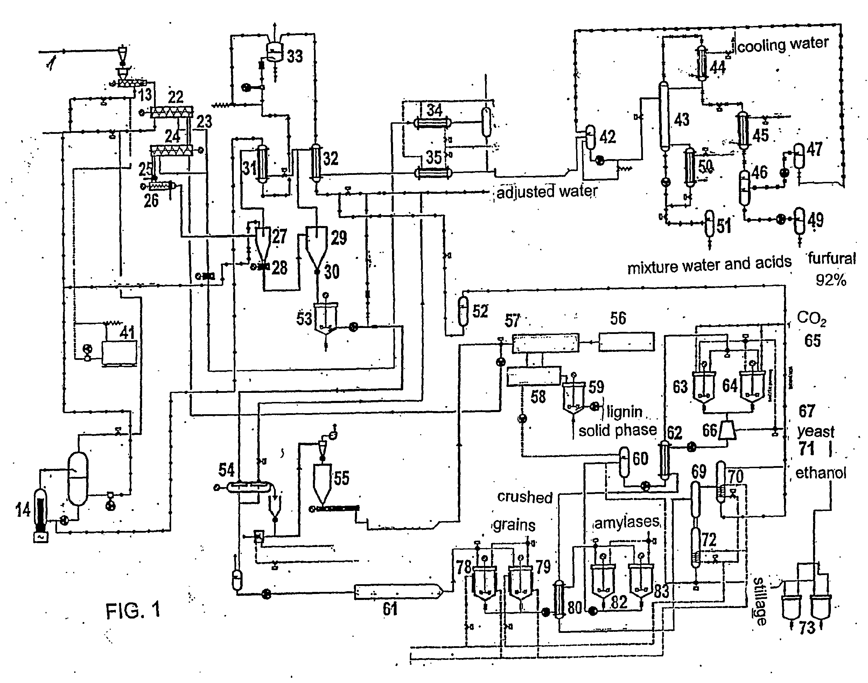

To figure 1
1. Belt conveyor
12. Processing tank
13. Filling unit
14. Electric boiler (gas boiler or solid fuels boiler)
22. First hydrolyser
23. Interconnecting pressure piping
24. Second hydrolyser
25. Overflow piping
26. High-pressure expansion slide valve
27. Medium-pressure expander
28. Medium-pressure expansion slide valve
29. Low-pressure expander
30. Rotary feeder
31. and 32. First exchangers (for the steam phase from the expansion)
33. Tank for process water
34. and 35. Second exchangers
42. Third tank (for Inlet furfural mixture)
43. First rectifying column
44. and 45.Third exchangers (for cooling the fural phase from the rectification)
46. Decanter
47. Fifth tank (for 8 % fural mixture)
49. Fourth tank (for 92 % fural mixture)
50. Exchanger (for cooling the mixture from the rectifying column)
51. Tank for the solution of mixture of acetic acid, formic acid and water
52. Sixth accumulative water tank (- part of the thermal cycle)
53. Stirred tank of the hydrolytic product
54. Separating device
55. Second tank (for unreacted solid lignocellulosic residues)
56. Device for preparation cellulase enzyme
57. Enzyme hydrolyser of cellulose to glucose
58. Separator of glucose and Ignin
59. Sixth tnk for Ignin
60. Seventh tank for preparation of the fermentative medium
61. First tank (for the solution of sugar hydrolysate)
62. Fifth exchanger
63. and 64. Fermenters
66. Yeast cell separator
67. Eighth tank for yeast cells
69. Distilling device
70. Second rectifying column
72. Evaporator
73. Ninth tanks (for stillage, yeast cells , biogas
78. and 79. Pressure reactor for starchliquefying
80. Fourth exchanger
82. and 83. Enzyme starch hydrolyser (for saccharifying)

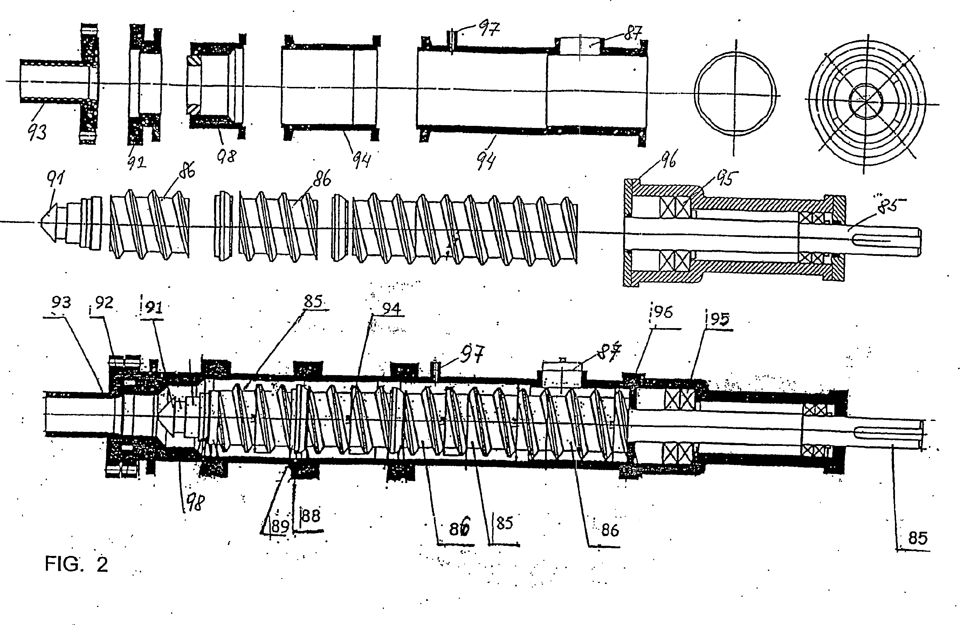

To figure 2
85. Shaft
86. Single-threaded conveyer worm
87. First opening (for raw materials)
88. Steam ring
89. Spacer
91. Mandrel
92. Output flange
93. Outlet filler with reducing part
94. Body
95. Bearing
96. Connecting board
97. Second opening
98. Head

### Examples of Execution

Figure 1 demonstrates the device for processing lignocellulosic and starch materials. It is continuous.

The device consists of a belt conveyor 1, connected to the worm pressure filling unit 13. The filling unit 13 is connected to the first hydrolyser 22, which is connected to the last, second hydrolyser 24. The first hydrolyser 22 is equipped with a feed piping, through which acid for the adjustment of the acidity in the first hydrolyser 22 is supplied.

There may be several hydrolysers which are depending on the processing capacity. Each of them is fitted with a driving conveyor worm and a connecting pressure piping between the hydrolysers for passing of the raw material.

The last second hydrolyser 24 is connected via the pressure overflow pipe 25 with a high-pressure expansion slide valve 26 to the medium-pressure expander 27. The lower part of the medium-pressure expander 27 is interconnected via the medium-pressure expansion slide valve 28 through the piping to the upper part of the atmospheric low-pressure expander 29, the lower part of the atmospheric low-pressure expander 29 is interconnected via the rotary feeder 30 through the piping to the stirred tank 53 of the hydrolytic product, which is further connected through the piping to the separating device 54, e. g. filtering press. Its first collecting part is interconnected to the first tank 61 for the solution of sugar hydrolysate and the second collecting part for the solid unreacted phase is interconnected to the second tank 55 for the unreacted solid lignocellulosic residues phase, the upper part of the medium-pressure expander 27 and the low-pressure expander 29 are interconnected through the piping to the first exchangers 31 and 32, second exchangers 34 and 35, and via the third tank 42 to the upper part of the first rectifying column 43, the upper part of the first rectifying column 43 is connected via the third exchangers 44 and 45 through the lower part of the decanter 46 to the fourth tank for furfural 49, the upper part of the decanter 46 is interconnected via the fifth tank 47 for the low-concentration furfural mixture (8%) back to the third tank 42.

The medium pressure expanders 27 and low pressure expanders 29 are shaped like cyclone separators and the medium-pressure expansion slide valve 28 tangentially enters the medium-pressure expander 27.

The second tank 55 for unreacted solid lignocellulosic residues is interconnected through a piping to the enzyme hydrolyser 57, which is interconnected to the device for preparation enzymes 56 and a separator 58 designed for the separation of glucose and lignin, the separator 58 is connected with the sixth tank 59 for lignin and with the seventh tank 60 for preparation of the fermentative medium, the first tank 61 for the solution of sugar hydrolysate from the hydrolysis is connected to the pressure reactors 78 and 79 for starchliquefying, which are equipped with a supply of grounded starch raw material. The pressure reactors 78 and 79 for starchliquefying are interconnected to the enzyme starch hydrolysers 82 and 83. which are further interconnected via the fourth exchanger 80 and the fifth exchanger 62 into the fermentors 63 and 64. Both fermentors are interconnected via the yeast cell separator 66 to the fourth heat exchanger 80, which is connected to the distilling device 69, which is interconnected to the evaporator 72 of the stillage and the second rectifying column 70. The distilling device 69, evaporator 72 and second rectifying column 70 are interconnected to the heating unit, the second rectifying column 70 in the area of the exhaust water is connected to the sixth accumulative water tank 52 for the process water heated in the first exchangers 32 and 33 to the boiler 14 of the heating unit.

Figure 2 presents the worm continuous filling unit 13. The continuous filling unit 13 consists of segments made up of the body 94 with single-threaded conveyer worms 86, positioned on the shaft 85. The set of segments is completed by the head 98, the inner geometrical shape of which is adjusted to the position of the mandrel 91 screwed into the end of the shaft 85. Between the worms there is at least one steam ring 88 and spacer 89, securing the steam ring 88 to the length of the whole worm set 86. The steam ring 88 widens conically on the entering side of the raw material. In the position of the steam ring 88 the inside part of the body 94 is fitted with the filler shaped like a thin annular ring 3 to 6 mm wide, the output flange 92 fitted with an outlet filler with the reducing part 93 leading to the first hydrolyser 22 is fastened to the head. The body 94 of one of the segments is fitted with the side first opening 87 for the intake of disintegrated raw material and with the second opening 97 for injection the process water feed. In front of the first worm on the shaft there is also fixed the connecting board 96 and bearings 95, the shaft is connected to the driving aggregate.

The disintegrated raw material is continuously fed into the input tank for the filling worm pressure filling u nit 13, consisting of several constructional parts. The worm transports the modified raw material into the second part, into which the process water is injected. The raw material proceeds through the spacer 89 and steam ring 88, where the raw material is compressed and heated as it passes from one section into the other. The raw material heated by this way is further transported by the worm 86 with the same diameter through the spacer 89 into another part with a connected single threaded worm 86. terminating with the last steam ring 88 and a mandrel 91. The pressure in the last section leading into the hydrolyser is approximately 1,5 to 3,2 MPa and the temperature 80 to 90 °C. The heated and partly-defibered raw material leads along the perimeter of the steam ring 88 and through the outlet filler 93 with the reducing part into the input part of the first hydrolyser 22.

The reacted suspension is carried from the last hydrolyser 24, through the pressure piping via the high-pressure expansion slide valve 26 into the first medium-pressure expander, in side of which is a pressure of 0,8 MPa, and a temperature of 170 °C. In the upper parts of the expanders there are the second and third piping for the exhaust of the vapour phase. The greater proportion of the furfural leavs in vapour portion, the condensing and latent heat of which is used to preheat the process water in the steam second exchangers 34 and 35. The hydrolysate is carried from the lower part of the medium-pressure expander 27 through the medium-pressure expansion slide valve 28, which is connected to the atmospheric low-pressure expander 29, which is connected through the rotary feeder 30 to the stirred tank 53 of the hydrolytic product. The steam phase from the low-pressure expander 29 is carried through the first exchangers 31 and 32 via the pressure piping into the third tank 42 for the inlet furfural mixture. The inert vapour phase containing furfural is carried from the upper part of the hydrolysers via the pressure piping and through the second and third high-pressure expansion slide valve 26 into the exchanger, from where the condensed inert phase is carried to the third tank 42 for the furfural mixture and acids. The furfural mixture in the third tank 42 is at a temperature of 30°C and is injected by a pump into the first rectifying column 43 from whose upper plates the furfural mixture is carried through the pressure piping via the exchanger into the decanter 46 from the lower part of which 92 % w/w furfural is pumped for expedition, and the upper part contains the water-furfural part with 8 % w/w furfural, which is pumped through a stainless piping into the upper part of the first rectifying column 43. The distilling residue from the first rectifying column 43 is pumped through a stainless piping into the tank 51 for the solution of mixture of acids and water.

The solution is led from the lower part of the atmospheric low-pressure expander 29 via the rotary feeder 30 through the piping into the mixing tank 53, from which the hydrolysed mixture is led through the piping to the separating device 54 - filtering press or centrifuge, in which the sugar solution from the fiberized lignocellulose phase is by pressing separated. The sugar solution is collected in the first tank 61 for the solution of sugar hydrolysate The fiberized lignocellulose phase is led through the piping into the enzyme hydrolyser 57, into which all cellulose enzymes are bringht. From the enzyme hydrolyser 57 the hydrolysed solid phase is led away through two branches with bypassing into the separator 58 of the glucose and lignin solution, from which the lignin is led into the sixth tank 59 for lignin and the glucose solution is drived into the seventh tank 60 for production of the fermentative medium. Alternatively, the second branch is interconnected into the thermo- pressure hydrolytic system to complete the hydrolysis.

After the hydrolysis is complete, the glucose solution is led from the first tank 61 into the stirred heated pressure reactor 78 and then into the enzyme starch hydrolyser 82, where the enzyme saccharifying of starch into glucose occurs.

The glucose solution is led through the piping and pressure pump into the seventh tank 60 for production of the fermentative medium, from which it proceeds through the pressure pump and heat exchanger into the fermentor 63. The fermentor 63 is interconnected with the yeast cell separator 66, from where the yeast cells return to the fermentor 63 and the ethanol solution is heated in the heat exchanger and is further led to the distilling device 69, which is interconnected to the evaporator 72 a nd the second rectifying column 70, or eventually to the device for the dehydration of ethanol. The concentrated stilllage is led away from the evaporator 72. Waste water from the ethanol rectification and furfural part are recycled for worming up in the heating unit for further use in the hydrolytic and rectifying process.

The waste water is returned to the process in the full extent, except of the water contained in moistened products and rinsing water. Phosphoric acid can be used expediently because its salts work as nutrients.

The advantage of the method and the device is particularly the fact that the process of hydrolytic fermentative technology processes all the input material into marketable products, such as furfural, lignin, ethanol, acetic acid, formic acid, carbon dioxide and stillage with yeast cells.

The advantage of the solution for the production of all of the aforementioned products is particularly the fact that the process occurs in one compact production unit and utilizes of lignocellulosic and starch materials and can completely process a farming product in a waste-free process. The only waste is the rinsing water.

The solution has the advantage that it offers the maximum possible saving of heat energy necessary for the individual processes.

The technical and technological core of the device is solution of the filling unit of the hydrolytic, decompressing and other devices allowing transportation of input materials and suspensions by the continuous piston movement in the hydrolysers, at the desired temperatures, pressures and the delay period in the hydrolytic part of the lignocellulosic materials with a continuous convergence to the starch processing. Particular advantage of the technology and the device is that the process of hydrolytic fermentative technology for the production of bioethanol is processionally resolved in one compact production unit and only renewable resources are processed. A device of this type can processes as a matter of fact the whole of the biomass without considerable waste.

The device is fully feasible in the conditions of medium engineering plants in the Czech Republic and does not depend on importation. The introduction of this technology enables - in a high utilization percentage of 75 to 85 % - the production of low-cost saccharide resources from lignocellulosic materials as the most important input material for biotechnological productions, with these further advantages:
a) revitalisation of agricultural communities, increased employment
b) advantages of utilization of ethanol produced according to the proposed technology
c) profitability
d) world competitiveness
e) reduction of imported sources of fuel (crude oil)
f) universality of the technology
g) negligible impact on the environment
h) utilization of renewable domestic resources
i) variability of resources
j) possibility of exporting new technologies and know-how

### Example No.1

This example has been chosen for processing of wheat frumentaceous straw, containing 7 to 10 % of water. Example No. 1 has been chosen for a commercial plant with a medium processing capacity of 1100 kg of straw per hour, that is 7200 tons of processed input raw material over 300 working days. The device in picture 1 contains 8 working sets: 01- Raw material preparation, 02- Heating circuit, 03- Hydrolysing part, 04- Separation of furfural and acids, 05- Separation of the solution of sugar and solid unreacted phase, 06- Enzyme hydrolysis of the unreacted cellulose. Hydrolysing and enzyme glucose merge into one sugar product, which is - at the selling price 6 to 7 K 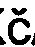 /kg - a well marketable product, or is further led into the tank for the preparation of the fermentative medium of set 07- for the production of ethanol; set 08 is designed for collection of carbon dioxide.

Set 01 Preparation of the input raw material. The main raw material in this stage is processing of wheat frumentaceous straw, but any other lignocellulosic raw material can also be used. The store of straw ensures the storage capacity for the preparation of crushed straw. The crushed straw is transported by the belt conveyor 1 to the pressure worm filling unit 13.

### Heating cycle - 02

Part of the process water is heated directly in the boiler 14, and is also used for generating steam. Pure demineralized water is processed in the boiler, which may be electric, gas, or for solid fuels. The steam detached from the two phase expansion provides heating of the modified water for completion of the system and is led, together with the exhaust water returning from the evaporator, via the first exchanger 32 where it is heated to 90 °C, proceeds further through the tank for process water 33, from where it proceeds via the first exchanger 31, in which it is heated to a temperature of 160 °C and is led to the boiler 14. Complementary water in the boiler is heated up from 160 °C to a temperature of 240 °C. The net heat consumption in the hydrolysing and fermentative set is 850 kW. The boiler continuously pumps water at a flow rate of 4500 to 6500 kg/hr. The boiler output is regulated according to the pressure in the separating vessel. The purpose of the separating vessel is to separate the liquid from the steam. The level of the liquid is maintained by the pump.

Hydrolysing set 03 for the production of furfural, organic acids, lignin, hydrolysing sugars.

Loose lignocellulosic material is brought to the processing tank 12 of the filling unit 13. The raw material proceeding through the spacer 89 and steam rings 88 where compression occurs is heated up to 80 to 90 °C. The partly defibrized material continuously enters to the counter pressure of the first hydrolyser 22 through the outlet filler 93 with reducing part and passes via the interconnecting pressure piping 23 into the second hydrolyser 24. The material after passing through the reducing part of the filling unit 13 slow disintegrates in the hydrolyser at temperatures of 195 - 235°C. 5000 kg of steam at a temperature of 235°C is dosed to the first hydrolyser 22 at the specified weight ratio. Acid at the weight ratio of 0,2 to 0,85 %, in relation to the suspension, is injected into the inlet piping in front of the first hydrolyser 22. The hydrolysed mixture proceeds to two-phase expansion. Vapour at a temperature of 170°C and a pressure of 0,8 MPa proceeds from the medium-pressure expander 27 to the first exchanger 31, where it preheats the process water from 90°C to a temperature of 160°C. The main reason for the double expansion is to improve the heat recuperation. Only the steam phase from the exchangers, not the sugar solution with the solid phase, is used for the recuperation. The solution drains by the medium-pressure expansion slide valve 28 into the cyclonic low-pressure expander 29. The vapour phase is mixed with the vapour phase of the medium-pressure 27 and low-pressure expander 29 and their condensing and latent heat is used in the first exchanger 32 to preheating the process water to 90°C. The heated process water proceeds from the first exchanger 32 into the tank for process water 33. The solution of sugar hydrolysate with the residues from the unreacted solid phase drains via the rotary feeder 30 to the stirred tank 53. The hydrolysing solution is discharged to another operating set 05 by the pump.

Separation of furaldehyde (set 04) from the steam phase of the expanders is solved in a continuous link-up to the set 03 of the hydrolysis. Furfural - the volatile components, are forced out from the hydrolysers first. The condensate of the steam and inert phase is led from the second exchangers 34 and 35 to the third tank 42 for the furfural mixture, where the mixture is cooled to 20 to 30°C and then is injected by the pump into the first rectifying column 43, from which the two streams leave. From the upper plates it is predominantly a solution of furfural, methanol and water, from the lower part it is a solution of acids and water. The stream of furfural is introduced through the third exchangers 44 and 45 into the decanter 46, where the cooled heterogeneous mixture is gravitationally separated into two layers. The lower layer contains approximately 92 % furfural solution in water and partly methanol. This layer is pumped off into the fourth tank 49 for 92% furfural, for further utilization as a product. The upper layer from the decanter 46 contains about 8 % furfural, furthermore methanol and water. This layer is injected back into the first rectifying column 43. The lower stream from the first rectifying column, 43, containing acids and water, is led away into the tank 51 for the solution of the acids mixture as a product or for further processing. This azeotropic mixture cannot be directly separated by rectification. It is necessary to use extraction first. If the third tank 42 for furfural mixture is not used, it is from, the point of view of furfural separation, more beneficial not to mix the output from the medium-pressure and atmospheric expanders and to direct each of them to a different plate of the first rectifying column 43.

Set 05 Separation solution of sugar and solid unreacted phase

The suspension of the mixture producted in stirred tank 53 is led by the pump to the filtering press - separating device 54, where the unreacted residues of cellulose and lignin are separated and led into the second tank 55 of the unreacted solid phase. The sugar solution is led to the first tank 61, from which it is pumped in to the seventh tank 60 for preparation of the fermentative medium.

Set 06 solves the enzyme hydrolysis of the unreacted phase.

Set 07 solves the processing of grains and starch.

The solution of hydrolytic glucose at a temeperature of approximately 80°C is alternatively pumped into the stired pressure reactors 78 and 79. Concurrently the crushed grains are fed into the reactors 78 and 79. The charge is heated to the temperature required for starchliquefying (about 100-110 °C) and after appropriate delay it is pumped through the fourth exchanger 80 into one of the enzyme starch hydrolysers 82 or 83, where - after adding amylases and adjusting the temperature and pH - the enzymie hydrolysis of starch into glucose occurs. The hydrolysed solution is pumped via the seventh tank 60 and through the fifth exchanger 62 by the pump into the fermentors 63 and 64.

Set 08 solves the fermentation and separation of ethanol occuring in consequention to the hydrolysing set and enzyme hydrolysis of starch. The glucose solution is modified to a concentration of 8 to 9 % w/w and after the addition of phosphates and trace elements Mg, Zn, and after sterilization it is further fermented.

Set 09 provides collection of CO₂ (the set is not elaborated in figures), at an output of approximately 100 to 150 kg/hr in the block medium-pressure arrangement, which ensures the separation of foam, purification and drying of CO₂ after the preceding compression in the oil-free compressors and subsequent forcing of the gaseous CO₂ phase into the tank for liquid CO₂, in which condensation by cooling occurs. It is possible to draw the liquid CO₂ or fill the pressure bottles with the liquid CO₂ from the tank. Should a higher output be required, the number of blocks is increased - the other components are not significantly changed.

Set 10 ensures the removal of the separated yeast cells in the eighth tank 67 and it carries these subproducts together with the stillage into the ninth tank 73. The stillage and yeast may be used as fodder.

The whole process has a high efficiency in industrial conditions. The distilling efficiency is about 99,5 %. By utilization of the service heat generated by hydrolysis and by recuperation of the heat consistently, it is not far from the parameters of the most up-to-date multipressure methods. Besides this, the device is simple and does not require intensive investment.

The linkage and relative simplicity of the fermentative, distilling and rectifying methods enable full automation and computerization of the process.

Comparison of the results of the efficiency of the individual products while processing only the lignocellulosic material (i) and together with the starch raw material (II).

| | |
|---|---|
| Input I | straw 1000 kg/hr |
| Input II | straw 1000 kg/hr. + 500 kg/hr. Starch grains |

Composition of the output streams:

| The main products of the hydrolysing solution in total | I | II |
|---|---|---|
| | 5 000 kg/hr | 5 000kg/hr |
| from it after modification: | | |
| furfural mixture 92% | 76 | 76 |
| mixture of acetic acid and formic acid | 30 | 30 |
| water | 944 | 944 |
| solid phase (lignin with unreacted cellulose) | 320 | 320 |
| solution of hydrolytic sugar and sugar from enzyme hydrolysis | 382 | 382 |
| amylolytic hydrolysis of starch into glucose | -- | 270 |
| Glucose in total | 382 | 652 |
| | | |

| Products from the fermentative and distilling process: | I | I II |
|---|---|---|
| | | |
| bio-ethanol liters | 254 | 442 |
| carbon dioxide CO₂ | 190 | 210 |
| stillage non concentrated | 960 | 1 100 |

The costs of production of bioethanol produced by the standard method are 2,5 to 3 times higher than the production of conventional engine fuels. The economic effectiveness of the production of bioethanol is affected by the costs of raw material, the size and configuration of the device, the costs of waste management and primarily energy costs. The by products provided by method of processing the lignocellulosic and starch material produced by the designed device increase the efficiency of the device and lower the costs of biethanol production.

### Industrial Applications

Ethanol with in a concentration of over 98 %w/e is the main product of the continuous process. There are several possible applications for its utilization.

It can be used as an additive for engine fuels and for ignition processes in general, in order to increase the fuel efficiency.

Part of the production can be used in the varnish and paint industry as a solvent, also in the chemical and foodstuffs industry.

Furhermore, the most important products are furaldehyde, lignin, acetic acid, formic acid, a limited amount of methanol, also carbon dioxide, stillage and yeast cells.

Lignin is a highly marketable material, especially popular as a filler component in the rubber-manufacturing industry as it has a very positive effect on the quality of the produced material (esp. for the production of tyres).

Furaldehyde, acetic acid and formid acid are commodities which sell sufficiently well on the chemical products market. Like lignin, they are highly marketable products, which are produced in the required quality and which significantly reduce the total costs of the operation.

Methanol is marketable on the chemical products market, and it can also be used in the engine fuel production industry.

Carbon dioxide is released in relatively considerable amounts and of very good quality (the practically pure output from the biological processes fulfill the requirements of the foodstuffs industry).

Stillage and yeast can be used in agro-industry.

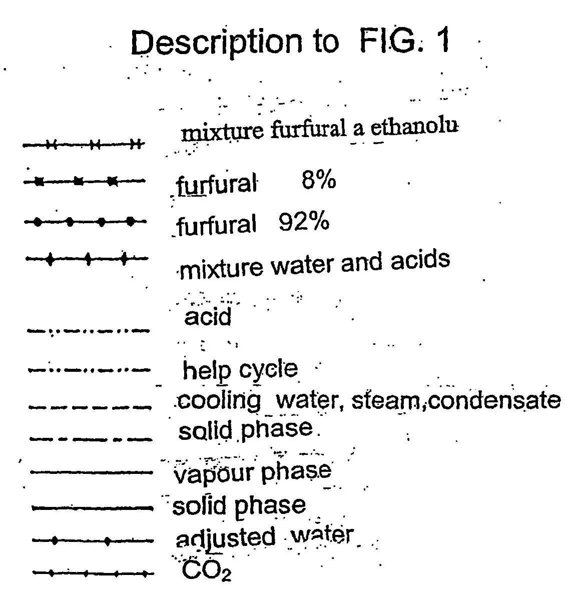

## Claims

1. A method for complex processing of lignocellulosic and starch materials where furfural, acidic acid and lignin are produced by continuous pressure hydrolysis, followed by two stages expansion, separation of the hydrolysate into the gaseous phase and solution of sugars, **characterized by** the fact that the disintegrated lignocellulosic raw material is continuously hydrolysed, the hydrolysed material is expanded in two stages when the vapour phase and hydrolysate solution are produced, in the vapour phase there is furfural, methanol and the acetic acid, the hydrolysate solution contains sugars, lignin with the residual cellulose and water, the hydrolysate solution is separated into the solution of sugar and solid unreacted phase by pressuring, the solid phase is exposed to cellulolytic enzymes, by the process of which soluble glucose and insoluble lignin are produced, the lignin is separated, the solution of glucose is added to the solution of sugar from the hydrolysis, to the solution of sugars is continuously added starch material and everything undergoes amylolytic hydrolysis, after which the solid particles are separated and returned to thermo-pressure hydrolysis, the solution of glucose is pumped for fermentation, where the glucose ferments to ethanol, the yeast cells are separated and ethanol is distilled off.

2. The method according to claim 1 , **characterized by** the fact that the disintegrated raw material is moistened in the weight ratio of 0,1 to 0,3 % w/w, during transportation the material is continuously mechanically heated to 80 to 90 °C, subsequently hydrolysed while simultaneously injecting steam containing 0,2 to 0,85% w/w of inorganic acid, in relation to the weight of the suspension, at a temperature of 190 to 235 °C, and a pressure of 1,5 to 3,2 MPa, the weight ratio of pressurized water to the solid base is 1:3,5 to 1:4.5, for a period of 9 to 12 minutes, when the hydrolysis proceeds during the simultaneous movement and balanced mixing and advance of the solid and liquid phase.

3. The method according to claim 1, **characterized by** the fact that the first expansion occurs at a temperature of 150 to 175°C and a pressure of 0,6 to 0,9 MPa, the greater proportion of the furfural leaves as the vapour portion, the condensing and latent heat of which is used in the exchanger for preheating the process water to a temperature of 160 °C and the second expansion occurs at a temperature of 105 to 110 °C and a pressure of 0,12 to 0,15 MPa, when the remaining furfural is separated from the liquid phase.

4. The method according to claims 1 to 3 **characterized by** the fact that for acceleration of the thermo-pressure hydrolysis an acid or acid-forming substance selected from a group consisting of phosphoric acid, hydrochloric acid, sulphuric acid, or superphosphate in a concentration of 0,3 to 0,85% of w/w is added, and the acid is mixed with steam in the piping before entering the hydrolyser.

5. The method according to claims 1 to 4 **characterized by** the fact that starch material, e. g. scrap, is dispersed in the glucose solution, which is adjusted to a pH of between 4 and 7 and to a temperature between 30 and 90 °C.

6. The method according to claims 1 to 5 **characterized by** the fact that the raw materials are heated by pressurized process water and that the unreacted solid phase is returned to the hydrolytic process.

7. The method according to claims 1 to 6 **characterized by** the fact that the heat energy of the hydrolysate is used for heating and starch liquefying of the suspension of starch materials in the solution of glucose and for heating the process water or steam.

8. The method according to claims 1 to 7 **characterized by** the fact that the heat energy obtained from the solution of glucose and by the concentration stillage is used for heating the mash.

9. The method according to claims 1 to 8 **characterized by** the fact that the heat energy of exhaust water is used to mixing the exhaust water with process water.

10. A device for carrying out the method according to claims 1 to 9, consisting of a crusher, filling unit and set of hydrolysers, of which the last one is interconnected via a medium-pressure expander and low-pressure expander to a stirred tank of hydrolytic product and the upper parts of the medium-pressure and low-pressure expanders are interconnected to the upper part of a rectifying column of furfural and to a furfural tank, **characterized by** the fact that the continuous pressure worm filling unit (13) consists of segments formed by a body (94) with single-threaded conveyer worms (86), positioned on a shaft (85), the set of segments is concluded by a head (98), inside which the geometric shape is adapted to the position of a mandrel (91), which is screwed into the end of the shaft (85), between the worms there is at least one steam ring (88) and spacer (89), the steam ring (88) widening conically on the entering side of the raw material, in the location of the steam ring (88) the inner part of the body (94) is fitted with a filler shaped like a thin annular ring 3 to 6 mm wide, an output flange (92) is attached to the head (98), the flange is fitted with an outlet filler (93) with the reducing part opening to the first hydrolyser (22), the body (94) of one of the segments is fitted with a side first opening (87) for the input of the disintegrated raw material and with a second opening (97) for injection of pressurized process water, furthermore, a connecting board (96) and bearings (95) are placed in front of the first worm on the shaft, the shaft (85) is connected to a driving propulsive aggregate, the first hydrolyser (22) is also equipped with a supply of steam with low concentrated acid, the first hydrolyser (22) is interconnected to at least one more hydrolyser, the last hydrolyser (24) is interconnected via a high-pressure expansion slide valve (26) to the medium-pressure expander (27), the lower part of which is interconnected via a medium-pressure expansion slide valve (28) into the upper part of the low-pressure expander (29), the lower part of the low-pressure expander (29) is interconnected via a rotary feeder (30) to the stirred tank (53) of the hydrolytic product, which is interconnected - via a separating device (54) - to a first tank (61) for the solution of sugar hydrolysate and with a second tank (55) for unreacted solid lignocellulosic residues, the upper part of the medium-pressure expander (27) and low-pressure expander (29) is interconnected with first exchangers (31) and (32), second exchangers (34) and (35), and through a third tank (42) into the upper part of a first rectifying column (43), the upper part of the first rectifiying column (43) is connected - via third exchangers (44) and (45) - through the lower part of a decanter (46) to the fourth tank for furfural (49), the upper part of the decanter (46) is interconnected through a fifth tank (47) for the low concentrated furfural mixture back to the third tank (42), the second tank (55) for unreacted solid lignocellulosic residues is interconnected to an enzyme hydrolyser (57), which is interconnected to a device (56) for the preparation of enzymes and a separator (58) designed for the separation of glucose and lignin, the separator (58) is connected to a sixth tank for lignin (59) and to a seventh tank (60) for the preparation of the fermentative medium, the first tank (61) for the solution of sugar hydrolysate is connected via a piping to pressure reactors (78) and (79) for starch liquefying, which are equipped with a supply of grounded starch raw material, the pressure reactors (78 and 79) are interconnected to enzyme starch hydrolysers (82) and (83), which are further interconnected through a fourth exchanger (80) and a fifth exchanger (62) into fermentors (63) and (64), both fermentors are connected via a yeast cell separator (66) to the fourth heat exchanger (80), which is connected to a distilling device (69) connected to an evaporator (72) and a second rectifying column (70) of ethanol, the distilling device (69), evaporator (72) and the second rectifying column (70) are connected to the heating unit, the second rectifying column (70) in the area of the exhaust water is interconnected to an eighth accumulative water tank (52) for process water to be heated in the first exchangers (32 and 33) for a boiler (14) of the heating unit.

11. The device according to claim 10 is **characterized by** the fact that the expanders (27) and (29) are shaped like cyclonic separators and the medium-pressure expansion slide valve (28) tangentially enters the medium pressure expander (27).

## Patentansprüche

1. Die Methode für die komplexe Verarbeitung der lignozelluloseartigen und Stärkematerialien, in denen Äthanol, Furfural, Essigsäure und Lignin durch die ununterbrochene Druckhydrolyse produziert werden, gefolgt von zwei Stadien Expansion, von der Trennung des Hydrolysats in die gasförmige Phase und die Lösung des Zuckers, **gekennzeichnet durch die Tatsache**, dass der aufgelöste lignozelluloseartige Rohstoff ununterbrochen hydrolysiert wird, das hydrolysierte Material in zwei Stadien expandiert, wobei die Dampfphase und die Hydrolysatlösung entstehen, in der Dampfphase sind Furfural, Methanol und die Essigsäure, das Hydrolysat enthält die Zucker, Lignin mit der Restzellulose und Wasser, das Hydrolysat wird **durch** Pressen in die Zuckerlösung und die feste unabreagierte Phase getrennt, man behandelt die feste Phase mit zellulolytischen Enzymen, **durch** dessen Prozess lösliche Glukose und unlösliches Lignin produziert werden, das Lignin wird getrennt ausgesetzt, die Glukoselösung wird in die Zuckerlösung von der Hydrolyse hinzugefügt, zur Zuckerlösung wird gleichzeitig ununterbrochen das Stärkematerial hinzugefügt und alles macht amylolytische Hydrolyse **durch**, nachdem werden die festen Partikel getrennt und zur Thermodruckhydrolyse zurückgebracht, die Glukoselösung wird für Gärung geschöpft, in der die Glukose zum Äthanol gärt, die Hefezellen werden getrennt und Äthanol wird weg destilliert.

2. Die Methode nach Anspruch 1, **gekennzeichnet durch** die Tatsache, dass der aufgelöste Rohstoff im Gewichtsverhältnis von 0,1 bis 0,3% des Gewichts befeuchtet ist, während des Transports ist das Material ununterbrochen auf 80 bis 90°C mechanisch erhitzt, nachher wird es gleichzeitig beim Injizieren von Dampf mit dem Inhalt 0,2 bis 0,85% des Gewichts von anorganischer Säure hydrolysiert, in Beziehung zu dem Gewicht der Suspension, bei der Temperatur von 190 bis 235°C und unter dem Druck von 1,5 bis 3,2 MPa, im Gewichtsverhältnis des Druckwassers zur Trockenmasse von 1: 3,5 bis 1: 4,5, für die Dauer von 9 bis 12 Minuten, wobei die Hydrolyse während der gleichzeitigen Bewegung und des ausgewogenen Mischens und des Fortschritts der festen und der flüssigen Phase abläuft.

3. Die Methode nach Anspruch 1, **gekennzeichnet durch die Tatsache**, dass die erste Expansion bei der Temperatur von 150 bis 175°C und unter dem Druck von 0,6 bis 0,9 MPa auftritt, der größere Anteil des Furfurals als Dampf-Abschnitts abgeht, dessen latente und Kondensationswärme im Austauscher für Vorwärmen des Verfahren-Wassers zu einer Temperatur von 160°C verwendet ist und die zweite Expansion tritt bei der Temperatur von 105 bis 110°C und unter dem Druck von 0,12 bis 0,15 MPa auf, wobei das verbleibende Furfural von der flüssigen Phase abgetrennt ist.

4. Die Methode nach Ansprüchen 1 bis 3, **gekennzeichnet durch die Tatsache**, dass für Beschleunigung der Thermodruckhydrolyse eine Säure oder eine säurebildende Substanz zugegeben wird, die von einer aus Phosphorsäure, einer Salzsäure, einer Schwefelsäure oder einem Superphosphat in Konzentration 0,3 bis 0,85% des Gewichts bestehenden Gruppe gewählt ist, die Säure ist mit Dampf in der Rohrleitung gemischt, bevor man den Hydrolyseapparat betritt.

5. Die Methode nach Ansprüchen 1 bis 4, **gekennzeichnet durch die Tatsache**, dass das Stärkematerial, z.B. Getreideschrott, in der Glukoselösung dispergiert, die auf ein pH zwischen von 4 und von 7 und auf eine Temperatur zwischen 30 und 90°C justiert wird.

6. Die Methode nach Ansprüchen 1 bis 5, **gekennzeichnet durch die Tatsache**, dass die Rohstoffe **durch** unter Druck gesetztes Verfahrenswasser geheizt werden und dass die unabreagierte feste Phase zum hydrolytischen Prozess zurückgebracht wird.

7. Die Methode nach Ansprüchen 1 bis 6, **gekennzeichnet durch die Tatsache**, dass die Wärmeenergie des Hydrolysats für Erwärmen und Verkleisterung der Suspension der Stärkematerialen in der Glukoselösung und für Erwärmen des Verfahrenswassers oder Verfahrensdampf verwendet ist.

8. Die Methode nach Ansprüchen 1 bis 7, **gekennzeichnet durch die Tatsache**, dass die von der Glukoselösung und der Verdickung der Schlempe gewonnene Wärmeenergie für Erwärmen der Maische verwendet ist.

9. Die Methode nach Ansprüchen 1 bis 8, **gekennzeichnet durch die Tatsache**, dass die Wärmeenergie des Absaugventilator-Wassers zum Mischen des Absaugventilator-Wassers mit Verfahrenswasser verwendet ist.

10. Die Vorrichtung für das Liefern der Methode nach Ansprüchen 1 bis 9, aus einem Zerkleinerer, einer Füllungseinheit und aus einem Satz von Hydrolyseapparaten bestehend, von denen der letzte über den Mitteldruckexpander und über den Unterdruckexpander mit gerührtem Tank des hydrolytischen Produkts verbunden ist und der obere Teil des Mitteldruck- und Unterdruckexpander zum oberen Teil der Rektifizierkolonne des Furfurals und zum Furfuraltank verbunden ist, **gekennzeichnet durch die Tatsache**, dass die ununterbrochene Druck-Schnecke-Füllung-Einheit (13) aus den Segmenten besteht, die **durch** den Körper (94) gebildet werden mit eingängigen, auf der Welle (85) positionierten Schnecken (86), der Satz der Segmente wird **durch** den Kopf abgeschlossen (98), dessen geometrische Innengestalt der Stellung des Dorns (91), das in das Ende der Welle (85) geschraubt ist, angepasst wird, zwischen den Schnecken ist mindestens ein Dampfschloss (88) und Abstandhalter (89), das Dampfschloss (88) erweitert sich konisch auf der Anlaufseite des Rohstoffs, an der Stelle des Dampfschloss (88) ist der innere Teil des Körpers (94) mit der Einlage in Form eines dünnen, 3 bis 6 Millimeter breiten Rings gesetzt, zum Kopf (98) ist der Ausgabeflansch (92) angeheftet, in dem die Ausgabeeinlage mit dem Übergangsteil (93) gesetzt ist, der in den ersten Hydrolyseapparat (22) mündet, der Körper (94) von einem der Segmente ist mit der ersten Öffnung der Seite (87) zuerst für die Eingabe des aufgelösten Rohstoffs und mit der zweiten Öffnung (97) für Einspritzung des Druck- Verfahrenswassers gesetzt, weiter sind vor der ersten Schnecke auf der Welle die Verbindungsplatte (96) und die Lager (95) gesetzt, die Welle (85) ist an das Antriebaggregat angeschlossen, der erste Hydrolyseapparat (22) ist zugleich mit einer Dampfzufuhr mit niedrig konzentrierter Säure gerüstet, der erste Hydrolyseapparat (22) ist an mindestens einen anderen Hydrolyseapparat angeschlossen, der letzte Hydrolyseapparat (24) ist über das Hochdruckschieberventil (26) zum Mitteldruckexpander (27) verbunden, dessen unterer Teil über das Mitteldruckschieberventil (28) in den oberen Teil des Unterdruckexpanders (29) verbunden ist, der untere Teil des Unterdruckexpanders (29) ist über den Drehzubringer (30) mit gerührtem Tank (53) des hydrolytischen Produkts verbunden, der über die Trennanlage (54) mit dem ersten Tank (61) von hydrolysierten Zuckern und mit dem zweiten Tank (55) für unabreagierte feste lignozelluloseartige Reste verbunden ist, der obere Teil des Mitteldruckexpanders (27) und des Unterdruckexpanders (29) sind mit den ersten Austauschern (31) und (32), den zweiten Austauschern (34) und (35), **durch** den dritten Tank (42) in den oberen Teil der ersten Rektifizierkolonne (43) verbunden, der obere Teil der ersten Rektifizierkolonne (43) ist über die dritten Austauscher (44) und (45) **durch** den unteren Teil des Dekantiergefäßes (46) mit dem vierten Tank für Furfural verbunden (49), der obere Teil des Dekantiergefäßes (46) ist **durch** den fünften Tank (47) mit niedrig konzentrierter Furfuralmischung zurück in den dritten Tank (42) verbunden, der zweite Tank (55) für unabreagierte feste Lignozellulosenreste ist mit dem Enzymhydrolyseapparat (57) verbunden, der mit der Anlage für Enzymvorbereitung (56) und mit dem für die Trennung von Glukose und Lignin bestimmten Separator (58) verbunden ist, der Separator (58) ist mit dem sechsten Tank für Lignin (59) und mit dem siebten Tank (60) für die Vorbereitung des Gärungsmediums verbunden, der erste Tank (61) für die Zuckerhydrolysatlösung ist über die Rohrleitung mit den Druckreaktoren (78) und (79) für die Stärkeverkleisterung verbunden, die mit der Zufuhr des zermahlten Stärkerohstoffs ausgestattet sind, die Druckreaktoren (78) und (79) sind mit den Enzymstärkehydrolyseapparaten (82) und (83) miteinander verbunden, die weiter über den vierten Austauscher (80) und den fünften Austauscher (62) in die Gärbütten (63) und (64) verbunden sind, beide Gärbütten sind **durch** den Hefeseparator (66) mit dem vierten Wärmeaustauscher (80) verbunden, der an den Destillationsapparat (69) angeschlossen ist, der mit dem Verdampfer (72) und mit der zweiten Rektifizierkolonne des Äthanols (70) verbunden ist, der Destillationsapparat (69), der Verdampfer (72) und die zweite Rektifizierkolonne (70) sind mit der Erwärmungseinheit verbunden, die Rektifizierkolonne ist im Bereich des Absaugventilator-Wassers mit dem achten Akkumulationswassertank (52) für Verfahrenswasser zur Erwärmung in den ersten Austauschern (32) und (33) in den Kessel (14) der Erwärmungseinheit verbunden.

11. Die Vorrichtung nach Anspruch 10, **gekennzeichnet durch die Tatsache**, dass die Expander (27) und (29) die Form der Zyklonabscheider haben und das Mitteldruckschieberventil (28) tangential den Mitteldruckexpander (27) betritt.

## Revendications

1. Le mode de traitement complexe de matériaux lignocellulosiques et amylacés, accompagné de la création de l'éthanol, du furfural, de l'acide acétique et de la lignine via l'hydrolyse continuelle sous pression, l'expansion bi-phasée suivante, la division de l'hydrolysat en phase gazeuse et solution de sucres, **caractéristique par le fait que** le matériau lignocellulosique désintégré est continuellement hydrolisé, le matériau hydrolisé expanse à deux degrés ce qui est accompagné par la création de la phase vapeur et de la solution d'hydrolysat, la phase vapeur comprend le furfural, le méthanol et l'acide acétique, l'hydrolysat comprend les sucres, la lignine avec la cellulose résiduaire et l'eau, l'hydrolysat se divise par le pressage en solution de sucres et en phase solide non modifiée, la phase solide est exposée aux enzymes cellulosiques ce qui est accompagné par la création de la glucose soluble et de la lignine non soluble qui se sépare, la solution de glucose s'ajoute dans la solution de sucres issus de l'hydrolyse, en même temps, on y ajoute de façon continuelle le matériau amylacé et le tout est soumis à l'hydrolyse amylolitique qui provoque la séparation des particules solides qui retournent à l'hydrolyse thermique sous pression, la solution glucosée est puisée pour la fermentation, les levures sont séparées après la fermentation de la glucose en éthanol qui est par la suite séparé par la distillation.

2. Le mode selon la revendication 1, caractéristique par le fait que la matière désintégrée est humidifiée en rapport de poids allant de 0,1 à 0,3 % du poids, pendant le transport, le matériau est continuellement réchauffé par voie mécanique à 80 - 90°C, ensuite, le matériau est hydrolisé et en même temps aspergé de vapeur qui contient de 0,2 à 0,85 % du poids de l'acide anorganique qui se rapporte au poids de la suspension, la température étant de 190 à 235°C et la pression étant de 1,5 à 3,2 Mpa, le rapport du poids eau sous pression/matière sèche étant de 1:3,5 - 1:4,5 pendant 9 à 12 minutes, l'hydrolyse a lieu alors que les phases solides et liquides sont mélangées de façon proportionnée et avancées.

3. Le mode selon la revendication 1, caractéristique par le fait que la première expansion se déroule sous la température allant de 150 à 175°C et sous la pression allant de 0,6 à 0,9 Mpa, la plus grande partie du furfural part avec la vapeur dont la chaleur de condensation et latente est utilisée dans l'échangeur pour le préchauffage de l'eau technologique à la température de 160°C et la deuxième expansion se déroule sous la température allant de 105 à 110°C et sous la pression de 0,12 à 0,15 Mpa où le furfural résiduel est séparé de la phase liquide.

4. Le mode selon les revendications 1 à 3, caractéristique par le fait que l'hydrolyse thermique sous pression est accélérée par un acide ou par une matière acidogène du groupe formé par l'acide phosphorique, chlorhydrique, sulfurique ou le superphosphate en concentration de 0,3 à 0,85 % du poids, l'acide est mélangé à la vapeur dans le conduit avant l'entrée dans l'hydrolyseur.

5. Le mode selon les revendications 1 à 4, caractéristique par le fait que le matériau amylacé, comme p.e. le grain de blé concassé, est dispergé dans la solution de glucose modifiée au pH allant de 4 à 7 et à la température entre 30 à 90°C.

6. Le mode selon les revendications 1 à 5, caractéristique par le fait que les matières premières sont préchauffées par l'eau technologique sous pression et la phase d'eau non modifiée revient au procès d'hydrolyse.

7. Le mode selon les revendications 1 à 6, caractéristique par le fait que l'énergie thermique de l'hydrolysat est utilisée pour le préchauffage et la gélatinisation de la suspension de matériaux féculeux dans la solution de glucose et pour le préchauffage de l'eau technologique ou de la vapeur.

8. Le mode selon les revendications 1 à 7, caractéristique par le fait que l'énergie thermique acquise à partir de la solution de glucose et de l'épaississement des vinasses est utilisée pour le préchauffage du macérat.

9. Le mode selon les revendications 1 à 8, caractéristique par le fait que l'énergie thermique de l'eau lutrée st utilisée lors du mélange de l'eau technologique.

10. Le dispositif servant à la réalisation selon les revendications 1 à 9 qui est composé d'un broyeur, d'unité de remplissage et d'un système d'hydrolyseurs, le dernier étant connecté via l'expandeur à pression moyenne et l'expandeur à basse pression avec le réservoir mélangé du produit d'hydrolyse et la partie supérieure de l'expandeur à pression moyenne et de l'expandeur à basse pression étant connectées à la partie supérieure de la colonne rectificative du furfural et du réservoir de furfural **caractéristique par le fait que** l'unité pression de remplissage continuel (13) consiste en segments formés par le corps (94) aux colimaçons simples (86) placés sur l'arbre (85), l'ensemble de segments étant achevé par une tête (98) dont la forme géométrique intérieure est adpatée à la position de l'aiguillon (91) placé à l'extrémité de l'arbre (85), entre les colimaçons, il y a au moins un cadenas à vapeur (88) et une cale déplacée (89), la naissance du cadenas à vapeur (88) est élargie de façon conique, au niveau du cadenas à vapeur (88), la partie intérieure du corps (94) est munie d'une pièce d'insertion en forme d'une couronne circulaire fine de 3 à 6 m, au niveau de la tête (98) est fixée une bride de sortie (92) dans laquelle est fixée la pièce d'insertion de sortie avec partie de passage (93) aboutissant au premier hydrolyseur (22), le corps (94) d'un des segments est muni d'une ouverture latérale (87) pour l'entrée de la matière désintégrée et d'une autre ouverture (97) pour le jet de l'eau technologique sous pression,ensuite, devant le premier colimaçon sur l'arbre, il y a une plaque de jonction (96) et des roulements (95), l'arbre (85) est lié à l'agrégat, le premier hydrolyseur (22) est interconnecté est en même temps muni de l'arrivée de la vapeur avec un acide peu concentré, le premier hydrolyseur (22) est connecté au moins avec un autre hydrolyseur, le dernier hydrolyseur (24) est connecté via la soupape haute pression (26) à l'expandeur pression moyenne (27) dont la partie inférieure est connectée via la soupape pression moyenne (28) à la partie supérieure de l'expandeur basse pression (29), la partie inférieure de l'expandeur (29) est connectée via le mécanisme rotatif d'alimentation (30) au réservoir mélangeur (53) du produit d'hydrolyse qui est connecté via le dispositif de séparation (54) au premier réservoir (61) de sucres obtenus par l'hydrolyse et au deuxième réservoir (55) destiné pour les résidus solides lignocellulosiques non réagis, la partie supérieure de l'expandeur pression moyenne (27) et de l'expandeur basse pression (29) est connectée aux premiers échangeurs (31) et (32), deuxièmes échangeurs (34) et (35) et via le troisième réservoir (42) à la partie supérieure de la première colonne rectificative (43), la partie supérieure de la première colonne rectificative (43) est connectée via les trois échangeurs (44) et (45) et via la partie inférieure du décanteur (46) au quatrième réservoir du furfural (49), la partie supérieure du décanteur (46) est connectée via le cinquième réservoir (47) de mélange furfural peu concentré au troisième réservoir (42), le deuxième réservoir (55) de résidus solides lignocellulosiques non réagis est connecté à l'hydrolyseur enzymatique (57) qui est connecté au dispositif destiné à la préparation d'enzymes (56) et au séparateur (58) destiné à la séparation de la glucose et de la lignine, le séparateur (58) est connecté au sixième réservoir de la lignine (59) et au septième réservoir (60) pour la préparation du milieu de fermentation, le premier réservoir (61) pour la solution de l'hydrolysat de sucre est connecté via le conduit aux réacteurs sous pression (78) et (79) destinés à la gélatinisation de l'amidon, ces derniers étant munis d'arrivées de l'amidon pulvérisé, les réacteurs sous pression (78 et 79) sont connectés aux hydrolyseurs d'amidon enzymatiques (82) et (83) qui sont ensuite connectés via le quatrième échangeur (80) et le cinquième échangeur (62) aux fermenteurs (63) et (64), les deux fermenteurs étant connectés via le séparateur (66) de levure au quatrième échangeur de chaleur (80) connecté à l'appareil de distillation (69) interconnecté à l'évaporateur (72) et à la deuxième colonne rectificative (70) de l'éthanol, l'appareil de distillation (69), l'évaporateur (72) et la deuxième colonne rectificative (70) sont connectés à l'unité de préchauffage, la colonne rectificative étant connectée au huitième réservoir d'accumulation d'eau (52) technologique destiné au préchauffage dans les premiers réservoirs (32) et (33) à la chaudière (14) de l'unité de chauffage.

11. Le dispositif selon la revendication 10 **caractéristique par le fait que** les expandeurs (27) et (29) ont la forme de séparateurs cycloniques et la soupape d'expansion pression moyenne (28) entre tangentiellement dans l'expandeur pression moyenne (27).
